# EUROPEAN PATENT SPECIFICATION

(11) **EP 4 462 163 B1**
(45) Date of publication and mention of the grant of the patent: **29.07.2026**
(21) Application number: 23826266.1
(22) Date of filing: 15.06.2023
(51) Int. Cl.: G02B 5/00, G02B 3/08, G02B 3/00, A61B 5/024, A61B 5/1455, G02B 5/04

(54) **FRESNEL FILM AND PREPARATION METHOD THEREFOR, OPTICAL DETECTION MODULE, AND WEARABLE DEVICE**
FRESNEL-FOLIE UND HERSTELLUNGSVERFAHREN DAFÜR, OPTISCHES DETEKTIONSMODUL UND WEARABLE-VORRICHTUNG
FILM DE FRESNEL ET SON PROCÉDÉ DE PRÉPARATION, MODULE DE DÉTECTION OPTIQUE ET DISPOSITIF HABITRONIQUE

(30) Priority: 22.06.2022 CN 202210715425
(43) Date of publication of application: 13.11.2024
(73) Proprietor: Huawei Technologies Co., Ltd., Shenzhen, Guangdong 518129 (CN)
(72) Inventor: TIAN, Yuqiang, Shenzhen, Guangdong 518129 (CN); YANG, Sulin, Shenzhen, Guangdong 518129 (CN); REN, Jingxuan, Shenzhen, Guangdong 518129 (CN); WANG, Xu, Shenzhen, Guangdong 518129 (CN)
(74) Representative: Gill Jennings & Every LLP
(86) International application number: PCT/CN2023/100425
(87) International publication number: WO 2023/246617

(56) References cited:
- CN-A- 107 219 573
- CN-A- 107 682 598
- CN-A- 111 936 892
- CN-A- 111 936 892
- CN-A- 113 069 079
- CN-A- 113 740 944
- CN-A- 113 866 934
- CN-A- 113 866 934
- CN-U- 213 934 271
- CN-U- 216 700 554
- CN-U- 216 724 540
- DE-A1- 102016 125 381
- JP-A- 2004 151 205
- JP-A- 2006 039 146
- JP-A- 2016 176 981

## Description

### CROSS-REFERENCE TO RELATED APPLICATIONS

This application claims priority to Chinese Patent Application No. 202210715425.5, filed with the China National Intellectual Property Administration on June 22, 2022 and entitled "FRESNEL MEMBRANE AND PRODUCTION METHOD THEREOF, OPTICAL DETECTION MODULE, AND WEARABLE DEVICE".

### TECHNICAL FIELD

This application relates to a Fresnel membrane, a Fresnel membrane production method, an optical detection module including the Fresnel membrane, and a wearable device.

### BACKGROUND

A wearable device is a portable device that can be worn on the body or integrated onto clothes or accessories of a user. With rapid development of electronic technologies, the wearable device can implement an increasing quantity of functions such as exercise monitoring, health monitoring, and sleep monitoring by detecting parameters of human bodies such as heart rates and blood oxygen saturation. The wearable device usually detects the parameters such as the heart rate and the blood oxygen saturation by using a PPG (Photoplethysmography, Photoplethysmography) pulse wave method, based on a principle of photoelectric detection that a blood volume changes with pulse beats. For example, the wearable device may emit light to a position of a human body at which the wearable device is worn, then detect an intensity of reflected light after the emitted light is absorbed by blood and tissues of the human body, trace changes in a blood vessel volume during a cardiac cycle to obtain a pulse waveform, and calculate the parameters such as the heart rate and the blood oxygen saturation based on the obtained pulse waveform.

A PPG detection module typically includes a light-emitting element, a photodiode, and a Fresnel membrane. The PPG detection module is visually occluded through an optical lens effect of the Fresnel membrane, improving optical efficiency of the PPG detection module. Light emitted by the light-emitting element (for example, an LED) passes through the Fresnel membrane and arrives at human tissues. After being reflected by the human tissues, the light passes through the Fresnel membrane again and is received by the photodiode. A heart rate and the like are detected based on received reflected optical signals. In an existing Fresnel membrane structure, ink is provided on surfaces of a Fresnel membrane that are opposite to each other, to separate an optical emit region from an optical receive region of the Fresnel membrane. However, the optical emit region and the optical receive region of the Fresnel membrane cannot be completely separated in this manner. As a result, a part of the light emitted by the LED is reflected by an interior that is of the Fresnel membrane and that corresponds to the ink region, and then directly received by the photodiode. To be specific, optical crosstalk exists between the optical emit region and the optical receive region. This part of optical signals are invalid signals, which reduce sensitivity of the optical detection module to bio-optical signals, increase power consumption of the device, and further reduce capabilities of detecting indicators such as the heart rate and the blood oxygen.

CN113866934A discloses relevant prior art.

### SUMMARY

A first aspect of this application provides a Fresnel membrane, including:
a substrate and Fresnel teeth located on a surface of the substrate, where
the Fresnel membrane further includes a through-hole that is provided in a thickness direction of the Fresnel membrane and runs through the substrate and the Fresnel teeth, the through-hole extends to form a closed circle, the Fresnel membrane further includes light-shielding ink that is stuffed in the through-hole, and the light-shielding ink divides the Fresnel membrane into two regions that are spaced apart from each other.

The light-shielding ink effectively divides the Fresnel membrane into two regions that are spaced apart from each other, avoiding occurrence of optical crosstalk between the two regions.

In an implementation of this application, a width of the light-shielding ink is 0.8 mm to 1.2 mm.

In an implementation of this application, the light-shielding ink divides the Fresnel membrane into an optical emit region and an optical receive region that are spaced apart from each other, and the light-shielding ink surrounds the optical emit region.

The light-shielding ink exists between the optical emit region and the optical receive region, to separate the optical emit region from the optical receive region. Because light cannot penetrate the light-shielding ink stuffed inside the Fresnel membrane, a case does not occur in which light emitted by a light-emitting element is reflected by an interior of the Fresnel membrane and then directly received by a photoelectric sensor.

In an implementation of this application, the through-hole includes a first groove and a second groove that communicate with each other, the first groove and the second groove are partially aligned in the thickness direction of the Fresnel membrane, each of the first groove and the second groove extends to form a closed circle, and the light-shielding ink is stuffed in the first groove and the second groove.

In an implementation of this application, the through-hole includes a first groove and a second groove that are arranged in the thickness direction of the Fresnel membrane and that communicate with each other, each of the first groove and the second groove extends to form a closed circle, the light-shielding ink is stuffed in the first groove, and a material obtained after a material of the substrate undergoes laser ablation is in the second groove.

In an implementation of this application, the substrate and the Fresnel teeth are integrally formed.

In an implementation of this application, the substrate and the Fresnel teeth are both transparent plastic materials.

A second aspect of this application provides a Fresnel membrane production method, including:
providing a transparent base membrane;
forming Fresnel teeth on a surface of the base membrane, so that the base membrane is formed into a substrate and the Fresnel teeth located on a surface of the substrate;
performing etching on the substrate and the Fresnel teeth, to form a through-hole that runs through the substrate and the Fresnel teeth in a thickness direction, where the through-hole extends to form a closed circle; and
stuffing light-shielding ink into the through-hole.

In an implementation of this application, the performing etching on the substrate and the Fresnel teeth to form a through-hole and the stuffing light-shielding ink into the through-hole include:
performing etching on the substrate and the Fresnel teeth to form a first groove, where the first groove extends to form a closed circle, and a depth of the first groove is less than a total thickness of the substrate and the Fresnel teeth;
stuffing the light-shielding ink into the first groove;
performing etching on the substrate and the Fresnel teeth to form a second groove, where the second groove is aligned with the first groove at least partially and extends to form a closed circle, and the second groove communicates with the first groove to form the through-hole; and
stuffing the light-shielding ink into the second groove.

In an implementation of this application, the method includes: performing embossing to form a first groove while performing embossing on the surface of the base membrane to form the Fresnel teeth, where the first groove extends to form a closed circle, and a depth of the first groove is less than a total thickness of the substrate and the Fresnel teeth;
stuffing the light-shielding ink into the first groove;
performing etching on the substrate and the Fresnel teeth to form a second groove, where the second groove is aligned with the first groove at least partially and extends to form a closed circle, and the second groove communicates with the first groove to form the through-hole; and
stuffing the light-shielding ink into the second groove.

In an implementation of this application, the performing etching on the substrate and the Fresnel teeth to form a through-hole and the stuffing light-shielding ink into the through-hole include:
performing etching on the substrate and the Fresnel teeth to form a first groove, where the first groove extends to form a closed circle, and a depth of the first groove is less than a total thickness of the substrate and the Fresnel teeth;
stuffing the light-shielding ink into the first groove; and
performing laser ablation on a region that is of the substrate and that is aligned with the light-shielding ink in the thickness direction of the substrate, to form a second groove, where a material obtained after a material of the substrate undergoes laser ablation is in the second groove, and the second groove is aligned with the first groove and extends to form a closed circle.

In an implementation of this application, the depth of the first groove is half the thickness of the substrate and the Fresnel teeth, and a depth of the second groove is half the thickness of the substrate and the Fresnel teeth.

In an implementation of this application, the performing etching on the substrate and the Fresnel teeth to form a through-hole and the stuffing light-shielding ink into the through-hole include:
performing etching on the substrate and the Fresnel teeth to form a first through-hole, where the first through-hole runs through the substrate and the Fresnel teeth;
stuffing the light-shielding ink into the first through-hole;
performing etching on the substrate and the Fresnel teeth to form a second through-hole, where the second through-hole runs through the substrate and the Fresnel teeth, and the second through-hole communicates with the first through-hole to form a closed circle; and
stuffing the light-shielding ink into the second through-hole.

In an implementation of this application, each of the first through-hole and the second through-hole extends to form a semicircular arc.

A third aspect of this application provides an optical detection module, including the Fresnel membrane according to the first aspect of this application, and a light-emitting element and a photoelectric sensor that are disposed on a side that is of the Fresnel membrane and that faces away from the Fresnel teeth, where the light-shielding ink divides the Fresnel membrane into an optical emit region and an optical receive region that are spaced apart from each other, the light-emitting element is disposed in alignment with the optical emit region of the Fresnel membrane, and the photoelectric sensor is disposed in alignment with the optical receive region of the Fresnel membrane.

The Fresnel membrane can effectively improve sensitivity of the optical detection module to bio-optical signals, and improve accuracy and capabilities of detecting indicators such as a heart rate and blood oxygen.

A fourth aspect of this application provides a wearable device, including the optical detection module according to the third aspect of this application.

### BRIEF DESCRIPTION OF DRAWINGS

FIG. 1 is a schematic sectional view of a Fresnel membrane in a conventional technology;
FIG. 2 is a schematic top view of a Fresnel membrane according to an embodiment of this application;
FIG. 3 is a schematic diagram of a section that is obtained after a Fresnel membrane in FIG. 2 is cut along a cutting plane line III-III;
FIG. 4 is a schematic diagram of an optical detection module according to an embodiment of this application;
FIG. 5 is a schematic diagram of a Fresnel membrane production process according to a first embodiment of this application;
FIG. 6 is a schematic diagram of a Fresnel membrane production process according to a second embodiment of this application;
FIG. 7 is a schematic diagram of a Fresnel membrane production process according to a third embodiment of this application;
FIG. 8 is a schematic diagram of a Fresnel membrane production process according to a fourth embodiment of this application; and
FIG. 9 is a schematic diagram of a Fresnel membrane production process according to a fifth embodiment of this application.

### List of reference numerals of main elements

| | |
|---|---|
| Fresnel membrane | 100, 300, 400, and 500 |
| Substrate | 10 |
| Fresnel teeth | 30 |
| Through-hole | 11 |
| Light-shielding ink | 50 |
| Optical emit region | 13 |
| Optical receive region | 15 |
| Optical detection module | 200 |
| Light-emitting element | 210 |
| Photoelectric sensor | 220 |
| Base | 230 |
| Foam | 240 |
| Base membrane | 110 |
| First groove | 111 |
| Second groove | 113 |
| First through-hole | 112 |
| Second through-hole | 114 |

### DESCRIPTION OF EMBODIMENTS

The following describes embodiments of this application with reference to the accompanying drawings in the embodiments of this application. Unless particularly specified, data range values described in this application all shall include end values.

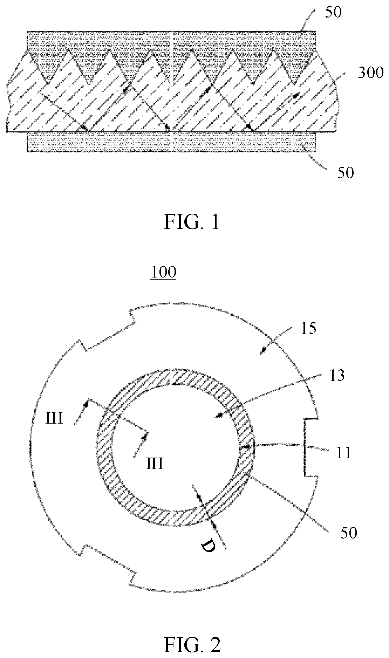

As shown in FIG. 1, for a Fresnel membrane 300 in an existing PPG optical detection module, light-shielding ink 50 is usually provided on both surfaces of the Fresnel membrane 300 that are opposite to each other, to separate an optical emit region from an optical receive region of the Fresnel membrane 300. However, a small part of light emitted by a light-emitting element is still reflected by an interior that is of the Fresnel membrane 300 and that corresponds to the light-shielding ink 50, and then directly received by a photodiode. As a result, the small part of light enters the photodiode directly without being reflected by skin of a human body. To be specific, optical crosstalk exists between the optical emit region and the optical receive region. This part of optical signals are invalid signals, which reduce sensitivity of the PPG optical detection module to bio-optical signals, and affect accuracy of detecting indicators such as a heart rate and blood oxygen.

This application provides a Fresnel membrane, with an optical emit region and an optical receive region of the Fresnel membrane effectively separated, avoiding occurrence of optical crosstalk between the optical emit region and the optical receive region.

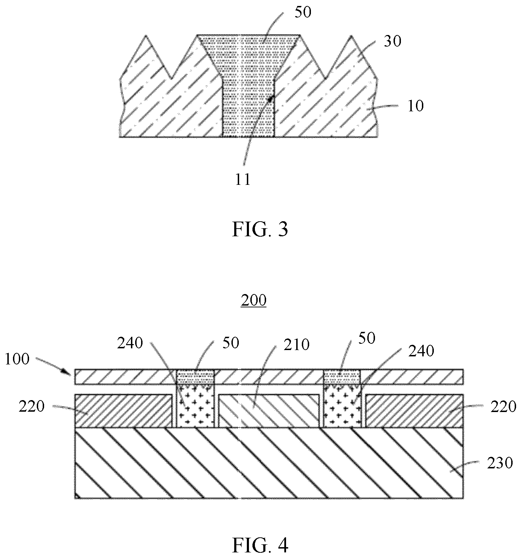

Refer to FIG. 2 and FIG. 3. A Fresnel membrane 100 in an embodiment of this application includes a substrate 10, and Fresnel teeth 30 that are located on a surface of the substrate 10. The Fresnel membrane 100 is provided with a through-hole 11 that runs through the substrate 10 and the Fresnel teeth 30 in a thickness direction of the Fresnel membrane 100. The through-hole 11 extends to form a closed circle. The Fresnel membrane 100 further includes light-shielding ink 50. The light-shielding ink 50 is stuffed in the through-hole 11. The light-shielding ink 50 divides the Fresnel membrane 100 into two regions that are spaced apart from each other. The two regions are an optical emit region 13 and an optical receive region 15. A surface that is of the substrate 10 and that faces away from the Fresnel teeth 30 is a smooth plane.

In this embodiment, the substrate 10 and the Fresnel teeth 30 are integrally formed. The substrate 10 and the Fresnel teeth 30 may be formed simultaneously by performing embossing on a transparent base membrane. In this embodiment, the substrate 10 and the Fresnel teeth 30 are both transparent plastic materials, for example, polyethylene terephthalate (Polyethylene Terephthalate, PET). In another embodiment, the substrate 10 and the Fresnel teeth 30 may alternatively be transparent glass. It may be understood that the substrate 10 and the Fresnel teeth 30 may alternatively not be integrally formed. For example, the Fresnel teeth 30 are additionally formed on the substrate 10. The Fresnel teeth 30 are configured to play a role of band-passing (reflection or refraction) for light in a specified spectral range.

In this embodiment, the light-shielding ink 50 surrounds the optical emit region 13, and the optical receive region 15 surrounds the light-shielding ink 50. To be specific, the optical emit region 13 is in the middle of the Fresnel membrane 100. The light-shielding ink 50 exists between the optical emit region 13 and the optical receive region 15, to separate the optical emit region 13 from the optical receive region 15. Because light cannot penetrate the light-shielding ink 50 stuffed inside the Fresnel membrane 100, a case does not occur in which light emitted by a light-emitting element is reflected by an interior of the Fresnel membrane 100 and then directly received by a photoelectric sensor.

In this embodiment, the Fresnel membrane 100 is a round sheet and thin. A thickness may vary from 0.1 mm to 0.15 mm depending on application. Corresponding, the through-hole 11 extends to form a ring. In another embodiment, the through-hole 11 may alternatively not be a ring/circle. For example, the through-hole 11 is a rectangular circle.

In this embodiment, a width D of the light-shielding ink 50 is from 0.8 mm to 1.2 mm. The width D is a width in a direction of a diameter of a ring formed by the light-shielding ink 50. The sufficient width D of the light-shielding ink 50 can ensure that optical crosstalk does not occur between the optical emit region 13 and the optical receive region 15.

In this embodiment, the Fresnel teeth 30 include multi-circle teeth that extend to form closed rings. The multi-circle teeth are concentric rings. The optical emit region 13 and the optical receive region 15 are both provided with the Fresnel teeth 30.

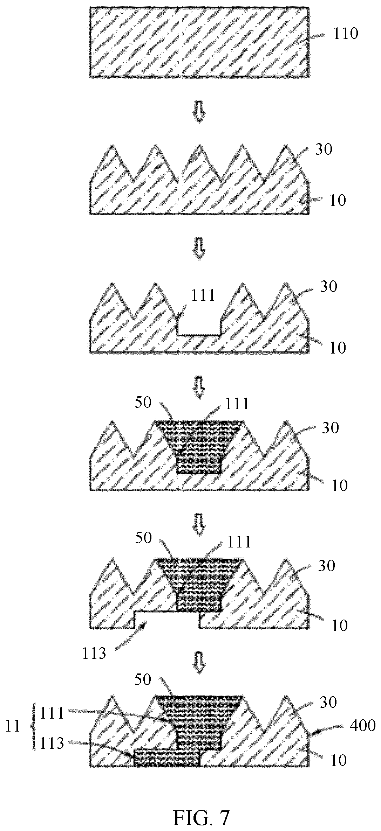

Refer to FIG. 7. A Fresnel membrane 400 in another embodiment is basically similar to the Fresnel membrane 100 in the foregoing embodiment, and also includes a substrate 10, Fresnel teeth 30 that are located on a surface of the substrate 10, a through-hole 11 that runs through the substrate 10 and the Fresnel teeth 30 in a thickness direction, and light-shielding ink 50 that is stuffed in the through-hole 11. The light-shielding ink 50 divides the Fresnel membrane 100 into two regions that are spaced apart from each other. In this embodiment, the through-hole 11 includes a first groove 111 and a second groove 113 that communicate with each other, the first groove 111 and the second groove 113 are partially aligned in the thickness direction of the Fresnel membrane 400, and the light-shielding ink 50 is stuffed in the first groove 111 and the second groove 113. Strength of the Fresnel membrane 400 is high.

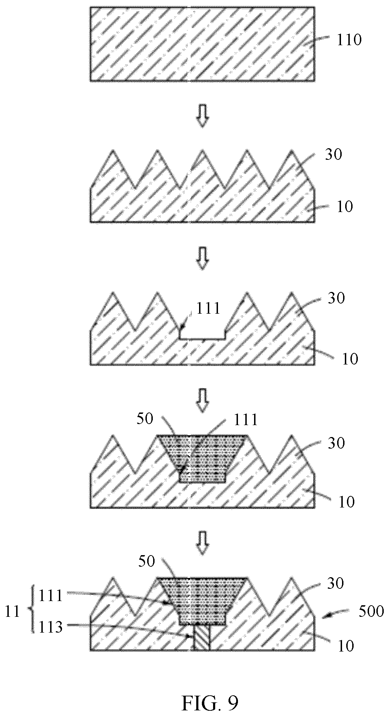

Refer to FIG. 9. A Fresnel membrane 500 in another embodiment is similar to the Fresnel membrane 400 in the foregoing embodiment. A through-hole 11 in the Fresnel membrane 500 also includes a first groove 111 and a second groove 113 that are arranged in a thickness direction of the Fresnel membrane 500 and that communicate with each other. In this embodiment, light-shielding ink 50 is stuffed only in the first groove 111, and a material obtained after a material of a substrate 10 undergoes laser ablation is in the second groove 113. After undergoing laser ablation, the material of the substrate 10 changes and is no longer transparent, implementing light shielding. Certainly, it may be understood that the second groove 113 may be alternatively considered absent but a case in which the material that is obtained after the substrate 10 undergoes laser ablation extends to form a closed circle and connects to the light-shielding ink 50. Strength of the Fresnel membrane 500 in this embodiment is high.

Refer to FIG. 4. An optical detection module 200 in an embodiment of this application includes the Fresnel membrane 100, and a light-emitting element 210 and a photoelectric sensor 220 that are disposed on a side that is of the Fresnel membrane 100 and that faces away from the Fresnel teeth 30. The light-emitting element 210 is disposed in alignment with the optical emit region 13 of the Fresnel membrane, so that light emitted by the light-emitting element 210 passes through the optical emit region 13 and is incident on a human body. The photoelectric sensor 220 is disposed in alignment with the optical receive region 15 of the Fresnel membrane 100, so that the light reflected by the human body passes through the optical receive region 15 and is received by the photoelectric sensor 220. The photoelectric sensor 220 may be a photodiode. It may be understood that the Fresnel membrane 100 may be alternatively replaced with the Fresnel membrane 400 in FIG. 7 or the Fresnel membrane 500 in FIG. 9.

The Fresnel membrane 100 can effectively improve sensitivity of the optical detection module 200 to bio-optical signals, and improve accuracy and capabilities of detecting indicators such as a heart rate and blood oxygen.

As shown in FIG. 4, in this embodiment, the optical detection module 200 further includes a base 230. The base 230 is an opaque material. The light-emitting element 210 and the photoelectric sensor 220 are both disposed on the base 230. The base 230 is configured to carry the light-emitting element 210 and the photoelectric sensor 220. The light-emitting element 210 and the photoelectric sensor 220 are located between the base 230 and the Fresnel membrane 100.

As shown in FIG. 4, further, foam 240 is disposed on the base 230. The foam 240 is supported between the base 230 and the Fresnel membrane 100, and surrounds the light-emitting element 210, that is, is located between the light-emitting element 210 and the photoelectric sensor 220. This can also avoid that light emitted by the light-emitting element 210 directly reaches a photosensitive surface of the photoelectric sensor 220.

This application further provides a wearable electronic device including the optical detection module. The wearable electronic device includes a housing. The optical detection module is disposed in the housing.

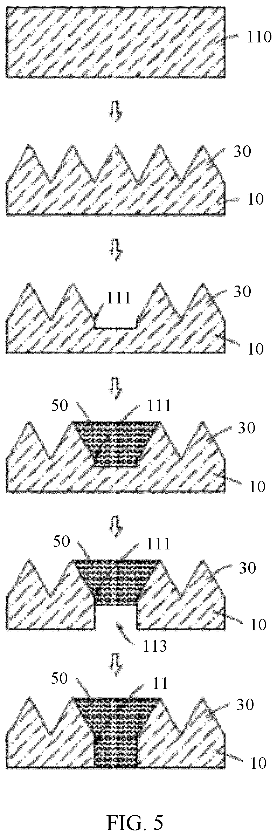

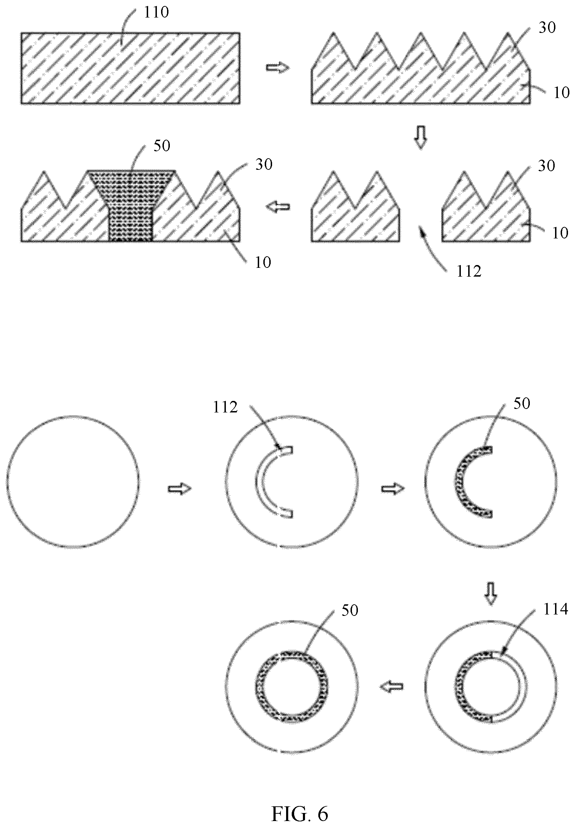

This application further provides a method for producing the Fresnel membrane 100. FIG. 5 and FIG. 6 illustrate two different production methods. The production method includes:
providing a transparent base membrane 110, as shown in FIG. 5 and FIG. 6;
performing embossing on a surface of the base membrane 110 to form Fresnel teeth 30, so that the base membrane 110 is formed into a substrate 10 and the Fresnel teeth 30 that are located on a surface of the substrate 10, as shown in FIG. 5 and FIG. 6;
performing etching on the substrate 10 and the Fresnel teeth 30, to form a through-hole 11 that runs through the substrate 10 and the Fresnel teeth 30, where the through-hole 11 extends to form a closed circle; and
stuffing light-shielding ink 50 into the through-hole 11.

In embodiments shown in FIG. 5 and FIG. 6, the base membrane 110 is a transparent plastic material. It may be understood that a manner in which the Fresnel teeth 30 are formed is not limited to embossing.

In the first embodiment, with reference to FIG. 5, the performing etching on the substrate 10 to form a through-hole 11 and the stuffing light-shielding ink 50 into the through-hole 11 include:
performing etching on the substrate 10 and the Fresnel teeth 30 to form a first groove 111, where the first groove 111 extends to form a closed circle, and a depth of the first groove 111 is less than a total thickness of the substrate 10 and the Fresnel teeth 30;
stuffing the light-shielding ink 50 into the first groove 111;
then performing etching on the substrate 10 and the Fresnel teeth 30 to form a second groove 113, where the second groove 113 is aligned with the first groove 111 and extends to form a closed circle, and the second groove 113 communicates with the first groove 111 to form the through-hole 11 that runs through the substrate 10 and the Fresnel teeth 30, that is, a depth of the second groove 113 is the total thickness of the substrate 10 and the Fresnel teeth 30 minus the depth of the first groove 111; and
stuffing the light-shielding ink 50 into the second groove 113.

In this embodiment, the depth of the first groove 111 is half the total thickness of the substrate 10 and the Fresnel teeth 30, and the depth of the second groove 113 is half the total thickness of the substrate 10 and the Fresnel teeth 30.

The through-hole 11 is formed by performing etching twice. For example, only half a depth of the through-hole 11 is formed by performing etching each time. If the through-hole 11 is formed by performing etching at one time, the Fresnel membrane 100 becomes two disconnected parts. This is not conducive to subsequent processing steps, because the Fresnel membrane 100 is a thin delicate product. Therefore, in this application, performing etching to form the through-hole 11 is carried out in two steps, to ensure that the Fresnel membrane 100 is always a whole throughout processing.

In the second embodiment, with reference to FIG. 6, the performing etching on the substrate 10 to form a through-hole 11 and the stuffing light-shielding ink 50 into the through-hole 11 include:
performing etching on the substrate 10 and the Fresnel teeth 30 to form a first through-hole 112, where the first through-hole 112 runs through the substrate 10 and the Fresnel teeth 30, and the first through-hole 112 extends to form an arc;
stuffing the light-shielding ink 50 into the first through-hole 112;
then performing etching on the substrate 10 and the Fresnel teeth 30 to form a second through-hole 114, where the second through-hole 114 runs through the substrate 10 and the Fresnel teeth 30, and the second through-hole 114 communicates with the first through-hole 112 to form a closed circle; and
stuffing the light-shielding ink 50 into the second through-hole 114.

In this embodiment, the first through-hole 112 extends to form a semicircular arc, and the second through-hole 114 extends to form a semicircular arc.

In this embodiment, the through-hole 11 is also formed by performing etching twice. For example, only half a length of the through-hole 11 is formed by performing etching each time. If the through-hole 11 is formed by performing etching at one time, the Fresnel membrane 100 becomes two disconnected parts. This is not conducive to subsequent processing steps, because the Fresnel membrane 100 is a thin delicate product.

FIG. 7 to FIG. 9 show three change embodiments of the production method in the first embodiment.

Refer to FIG. 7. A production method in a third embodiment is basically the same as the production method in the first embodiment shown in FIG. 5. A through-hole 11 is also formed by performing etching twice. After etching is performed for the first time, a first groove 111 is formed, and then light-shielding ink 50 is stuffed. After etching is performed for the second time, a second groove 113 is formed, and then light-shielding ink 50 is stuffed. A difference between the two embodiments is as follows: In this embodiment, the first groove 111 and the second groove 113 are partially aligned (not completely aligned) in a thickness direction of a substrate 10. In this way, the light-shielding ink 50 in the first groove 111 and the light-shielding ink 50 in the second groove 113 blend together. In the first embodiment, the first groove 111 and the second groove 113 are completely aligned in a thickness direction of the substrate 10. Strength of the Fresnel membrane 400 produced using the production method in the third embodiment is higher than strength of the Fresnel membrane 100 produced using the production method in the first embodiment to some extent.

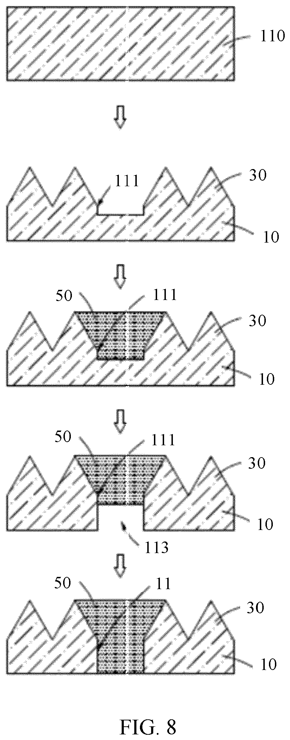

Refer to FIG. 8. A production method in a fourth embodiment is also basically the same as the production method in the first embodiment shown in FIG. 5. A difference between the two embodiments is as follows: In this embodiment, a first groove 111 is not formed by performing etching. Instead, the first groove 111 is formed by performing embossing while embossing is performed on a surface of a base membrane 110 to form Fresnel teeth 30. In this way, when the production method in the fourth embodiment is compared with the production method in the first embodiment, an etching step is saved, helping simplify a production process.

Refer to FIG. 9. A production method in a fifth embodiment is also basically the same as the production method in the first embodiment shown in FIG. 5. A difference between the two embodiments is as follows: In this embodiment, after etching is performed to form a first groove 111 and light-shielding ink 50 is stuffed, etching is not performed to form a second groove 113. Instead, laser ablation is performed on a region that is of a substrate 10 and that is aligned with the light-shielding ink 50, so that a material of the part of the substrate 10 melts and changes in properties (no longer transparent, implementing light shielding). This is equivalent to that laser ablation is performed to form the second groove 113 that is aligned with the first groove 111. A material obtained after the material of the substrate 10 undergoes laser ablation is in the second groove 113. Compared with the production method in the first embodiment, the production method in the fifth embodiment uses a simpler process. In addition, strength of the Fresnel membrane 500 produced using the production method in the fifth embodiment is high.

It should be noted that the foregoing descriptions are merely specific implementations of this application, but the protection scope of this application is not limited thereto. In a case of no conflict, the implementations of this application and the features in the implementations may be mutually combined. Therefore, the protection scope of this application shall be subject to the protection scope of the claims.

## Claims

1. A Fresnel membrane (100, 300, 400, 500), comprising:
a substrate (10) and Fresnel teeth (30) located on a surface of the substrate, wherein
the Fresnel membrane further comprises a through-hole (11) that is provided in a thickness direction of the Fresnel membrane and runs through the substrate and the Fresnel teeth, the through-hole extends to form a closed circle, the Fresnel membrane further comprises light-shielding ink (50)
that is stuffed in the through-hole, and the light-shielding ink divides the Fresnel membrane into two regions (13, 15) that are spaced apart from each other.

2. The Fresnel membrane according to claim 1, wherein the light-shielding ink extends by a width of 0.8 mm to 1.2 mm.

3. The Fresnel membrane according to claim 1 or 2, wherein the light-shielding ink divides the Fresnel membrane into an optical emit region and an optical receive region that are spaced apart from each other, and the light-shielding ink surrounds the optical emit region.

4. The Fresnel membrane according to any one of claims 1 to 3, wherein the through-hole comprises a first groove and a second groove that communicate with each other, the first groove and the second groove are partially aligned in the thickness direction of the Fresnel membrane, each of the first groove and the second groove extends to form a closed circle, and the light-shielding ink is stuffed in the first groove and the second groove.

5. The Fresnel membrane according to any one of claims 1 to 3, wherein the through-hole comprises a first groove and a second groove that are arranged in the thickness direction of the Fresnel membrane and that communicate with each other, each of the first groove and the second groove extends to form a closed circle, the light-shielding ink is stuffed in the first groove, and a material obtained after a material of the substrate undergoes laser ablation is in the second groove.

6. The Fresnel membrane according to any one of claims 1 to 5, wherein the substrate and the Fresnel teeth are integrally formed.

7. The Fresnel membrane according to any one of claims 1 to 6, wherein the substrate and the Fresnel teeth are both transparent plastic materials.

8. A Fresnel membrane (100, 300, 400, 500) production method, comprising:
providing a transparent base membrane (110);
forming Fresnel teeth (30) on a surface of the transparent base membrane, so that the transparent base membrane is formed into a substrate (10) and the Fresnel teeth located on a surface of the substrate:
performing etching on the substrate and the Fresnel teeth, to form a through-hole (11) that runs through the substrate and the Fresnel teeth in a thickness direction, wherein the through-hole extends to form a closed circle; and
stuffing light-shielding ink (50) into the through-hole.

9. The Fresnel membrane production method according to claim 8, wherein
the performing etching on the substrate and the Fresnel teeth to form a through-hole and the stuffing light-shielding ink into the through-hole comprise:
performing etching on the substrate and the Fresnel teeth to form a first groove, wherein the first groove extends to form a closed circle, and a depth of the first groove is less than a total thickness of the substrate and the Fresnel teeth;
stuffing the light-shielding ink into the first groove;
performing etching on the substrate and the Fresnel teeth to form a second groove, wherein the second groove is aligned with the first groove at least partially and extends to form a closed circle, and the second groove communicates with the first groove to form the through-hole; and
stuffing the light-shielding ink into the second groove.

10. The Fresnel membrane production method according to claim 8, comprising: performing embossing to form a first groove while performing embossing on the surface of the base membrane to form the Fresnel teeth, wherein the first groove extends to form a closed circle, and a depth of the first groove is less than a total thickness of the substrate and the Fresnel teeth;
stuffing the light-shielding ink into the first groove;
performing etching on the substrate and the Fresnel teeth to form a second groove, wherein the second groove is aligned with the first groove at least partially and extends to form a closed circle, and the second groove communicates with the first groove to form the through-hole; and
stuffing the light-shielding ink into the second groove.

11. The Fresnel membrane production method according to claim 8, wherein the performing etching on the substrate and the Fresnel teeth to form a through-hole and the stuffing light-shielding ink into the through-hole comprise:
performing etching on the substrate and the Fresnel teeth to form a first groove, wherein the first groove extends to form a closed circle, and a depth of the first groove is less than a total thickness of the substrate and the Fresnel teeth;
stuffing the light-shielding ink into the first groove; and
performing laser ablation on a region that is of the substrate and that is aligned with the light-shielding ink in the thickness direction of the substrate, to form a second groove, wherein a material obtained after a material of the substrate undergoes laser ablation is in the second groove, and the second groove is aligned with the first groove and extends to form a closed circle.

12. The Fresnel membrane production method according to any one of claims 9 to 11, wherein the depth of the first groove is half the thickness of the substrate and the Fresnel teeth, and a depth of the second groove is half the thickness of the substrate and the Fresnel teeth.

13. The Fresnel membrane production method according to claim 8, wherein the performing etching on the substrate and the Fresnel teeth to form a through-hole and the stuffing light-shielding ink into the through-hole comprise:
performing etching on the substrate and the Fresnel teeth to form a first through-hole, wherein the first through-hole runs through the substrate and the Fresnel teeth;
stuffing the light-shielding ink into the first through-hole;
performing etching on the substrate and the Fresnel teeth to form a second through-hole, wherein the second through-hole runs through the substrate and the Fresnel teeth, and the second through-hole communicates with the first through-hole to form a closed circle; and
stuffing the light-shielding ink into the second through-hole.

14. An optical detection module, comprising the Fresnel membrane according to any one of claims 1 to 7, and a light-emitting element and a photoelectric sensor that are disposed on a side that is of the Fresnel membrane and that faces away from the Fresnel teeth, wherein the light-shielding ink divides the Fresnel membrane into an optical emit region and an optical receive region that are spaced apart from each other, the light-emitting element is disposed in alignment with the optical emit region of the Fresnel membrane, and the photoelectric sensor is disposed in alignment with the optical receive region of the Fresnel membrane.

15. A wearable device, comprising the optical detection module according to claim 14.

## Patentansprüche

1. Fresnel-Membran (100, 300, 400, 500), umfassend:
ein Substrat (10) und Fresnel-Zähne (30), die sich auf einer Oberfläche des Substrats befinden, wobei die Fresnel-Membran ferner ein Durchgangsloch (11) umfasst, das in einer Dickenrichtung der Fresnel-Membran bereitgestellt ist und durch das Substrat und die Fresnel-Zähne verläuft, sich das Durchgangsloch erstreckt, um einen geschlossenen Kreis zu bilden, die Fresnel-Membran ferner eine Lichtabschirmtinte (50) umfasst, die in das Durchgangsloch gefüllt ist, und die Lichtabschirmtinte die Fresnel-Membran in zwei Bereiche (13, 15) unterteilt, die voneinander beabstandet sind.

2. Fresnel-Membran nach Anspruch 1, wobei sich die Lichtabschirmtinte über eine Breite von 0,8 mm bis 1,2 mm erstreckt.

3. Fresnel-Membran nach Anspruch 1 oder 2, wobei die Lichtabschirmtinte die Fresnel-Membran in einen optischen Emissionsbereich und einen optischen Empfangsbereich unterteilt, die voneinander beabstandet sind, und die Lichtabschirmtinte den optischen Emissionsbereich umgibt.

4. Fresnel-Membran nach einem der Ansprüche 1 bis 3, wobei das Durchgangsloch eine erste Nut und eine zweite Nut umfasst, die miteinander in Verbindung stehen, die erste Nut und die zweite Nut teilweise in der Dickenrichtung der Fresnel-Membran ausgerichtet sind, sich jede der ersten Nut und der zweiten Nut erstreckt, um einen geschlossenen Kreis zu bilden, und die Lichtabschirmtinte in die erste Nut und die zweite Nut gefüllt ist.

5. Fresnel-Membran nach einem der Ansprüche 1 bis 3, wobei das Durchgangsloch eine erste Nut und eine zweite Nut umfasst, die in der Dickenrichtung der Fresnel-Membran angeordnet sind und miteinander in Verbindung stehen, sich jede der ersten und der zweiten Nut erstreckt, um einen geschlossenen Kreis zu bilden, die Lichtabschirmtinte in die erste Nut gefüllt ist und sich in der zweiten Nut ein Material befindet, das erlangt wird, nachdem ein Material des Substrats einer Laserablation unterzogen wurde.

6. Fresnel-Membran nach einem der Ansprüche 1 bis 5, wobei das Substrat und die Fresnel-Zähne einstückig ausgebildet sind.

7. Fresnel-Membran nach einem der Ansprüche 1 bis 6, wobei das Substrat und die Fresnel-Zähne beide aus transparentem Kunststoff bestehen.

8. Verfahren zur Herstellung von Fresnel-Membranen (100, 300, 400, 500), umfassend:
Bereitstellen einer transparenten Basismembran (110);
Bilden von Fresnel-Zähnen (30) auf einer Oberfläche der transparenten Basismembran, sodass die transparente Basismembran zu einem Substrat (10) gebildet wird und sich die Fresnel-Zähne auf einer Oberfläche des Substrats befinden;
Durchführen von Ätzen an dem Substrat und den Fresnel-Zähnen, um ein Durchgangsloch (11) zu bilden, das in einer Dickenrichtung durch das Substrat und die Fresnel-Zähne verläuft, wobei sich das Durchgangsloch erstreckt, um einen geschlossenen Kreis zu bilden; und
Füllen von Lichtabschirmtinte (50) in das Durchgangsloch.

9. Verfahren zur Herstellung von Fresnel-Membranen nach Anspruch 8, wobei
das Durchführen des Ätzens an dem Substrat und den Fresnel-Zähnen, um ein Durchgangsloch zu bilden, und das Füllen von Lichtabschirmtinte in das Durchgangsloch Folgendes umfassen:
Durchführen von Ätzen an dem Substrat und den Fresnel-Zähnen, um eine erste Nut zu bilden, wobei sich die erste Nut erstreckt, um einen geschlossenen Kreis zu bilden, und eine Tiefe der ersten Nut geringer ist als eine Gesamtdicke des Substrats und der Fresnel-Zähne;
Füllen der Lichtabschirmtinte in die erste Nut;
Durchführen von Ätzen an dem Substrat und den Fresnel-Zähnen, um eine zweite Nut zu bilden, wobei die zweite Nut mindestens teilweise mit der ersten Nut ausgerichtet ist und sich erstreckt, um einen geschlossenen Kreis zu bilden, und die zweite Nut mit der ersten Nut in Verbindung steht, um das Durchgangsloch zu bilden; und
Füllen der Lichtabschirmtinte in die zweite Nut.

10. Verfahren zur Herstellung von Fresnel-Membranen nach Anspruch 8, umfassend: Durchführen von Prägen, um eine erste Nut zu bilden, bei gleichzeitigem Durchführen von Prägen an der Oberfläche der Basismembran, um die Fresnel-Zähne zu bilden, wobei sich die erste Nut erstreckt, um einen geschlossenen Kreis zu bilden, und eine Tiefe der ersten Nut geringer ist als eine Gesamtdicke des Substrats und der Fresnel-Zähne;
Füllen der Lichtabschirmtinte in die erste Nut;
Durchführen von Ätzen an dem Substrat und den Fresnel-Zähnen, um eine zweite Nut zu bilden, wobei die zweite Nut mindestens teilweise mit der ersten Nut ausgerichtet ist und sich erstreckt, um einen geschlossenen Kreis zu bilden, und die zweite Nut mit der ersten Nut in Verbindung steht, um das Durchgangsloch zu bilden; und
Füllen der Lichtabschirmtinte in die zweite Nut.

11. Verfahren zur Herstellung von Fresnel-Membranen nach Anspruch 8, wobei das Durchführen von Ätzen an dem Substrat und den Fresnel-Zähnen, um ein Durchgangsloch zu bilden, und das Füllen von Lichtabschirmtinte in das Durchgangsloch Folgendes umfassen:
Durchführen von Ätzen an dem Substrat und den Fresnel-Zähnen, um eine erste Nut zu bilden, wobei sich die erste Nut erstreckt, um einen geschlossenen Kreis zu bilden, und eine Tiefe der ersten Nut geringer ist als eine Gesamtdicke des Substrats und der Fresnel-Zähne;
Füllen der Lichtabschirmtinte in die erste Nut; und
Durchführen von Laserablation an einem Bereich, der zu dem Substrat gehört und der mit der Lichtabschirmtinte in der Dickenrichtung des Substrats ausgerichtet ist, um eine zweite Nut zu bilden, wobei sich in der zweiten Nut ein Material befindet, das erlangt wird, nachdem ein Material des Substrats einer Laserablation unterzogen wurde, und die zweite Nut mit der ersten Nut ausgerichtet ist und sich erstreckt, um einen geschlossenen Kreis zu bilden.

12. Verfahren zur Herstellung von Fresnel-Membranen nach einem der Ansprüche 9 bis 11, wobei die Tiefe der ersten Nut die Hälfte der Dicke des Substrats und der Fresnel-Zähne beträgt und eine Tiefe der zweiten Nut die Hälfte der Dicke des Substrats und der Fresnel-Zähne beträgt.

13. Verfahren zur Herstellung von Fresnel-Membranen nach Anspruch 8, wobei das Durchführen von Ätzen an dem Substrat und den Fresnel-Zähnen, um ein Durchgangsloch zu bilden, und das Füllen von Lichtabschirmtinte in das Durchgangsloch Folgendes umfassen:
Durchführen von Ätzen an dem Substrat und den Fresnel-Zähnen, um ein erstes Durchgangsloch zu bilden, wobei das erste Durchgangsloch durch das Substrat und die Fresnel-Zähne verläuft;
Füllen der Lichtabschirmtinte in das erste Durchgangsloch;
Durchführen von Ätzen an dem Substrat und den Fresnel-Zähnen, um ein zweites Durchgangsloch zu bilden, wobei das zweite Durchgangsloch durch das Substrat und die Fresnel-Zähne verläuft und das zweite Durchgangsloch mit dem ersten Durchgangsloch in Verbindung steht, um einen geschlossenen Kreis zu bilden; und
Füllen der Lichtabschirmtinte in das zweite Durchgangsloch.

14. Optisches Detektionsmodul, umfassend die Fresnel-Membran nach einem der Ansprüche 1 bis 7 und ein Leuchtelement und einen fotoelektrischen Sensor, die auf einer Seite angeordnet sind, welche zu der Fresnel-Membran gehört und welche von den Fresnel-Zähnen abgewandt ist, wobei die Lichtabschirmtinte die Fresnel-Membran in einen optischen Emissionsbereich und einen optischen Empfangsbereich unterteilt, die voneinander beabstandet sind, das Leuchtelement in Ausrichtung mit dem optischen Emissionsbereich der Fresnel-Membran angeordnet ist und der fotoelektrische Sensor in Ausrichtung mit dem optischen Empfangsbereich der Fresnel-Membran angeordnet ist.

15. Wearable-Vorrichtung, umfassend das optische Detektionsmodul nach Anspruch 14.

## Revendications

1. Membrane de Fresnel (100, 300, 400, 500), comprenant :
un substrat (10) et des dents de Fresnel (30) situées sur une surface du substrat, dans laquelle la membrane de Fresnel comprend en outre un trou traversant (11) qui est prévu dans une direction d'épaisseur de la membrane de Fresnel et traverse le substrat et les dents de Fresnel, le trou traversant s'étend pour former un cercle fermé, la membrane de Fresnel comprend en outre une encre de protection contre la lumière (50) qui est remplie dans le trou traversant, et l'encre de protection contre la lumière divise la membrane de Fresnel en deux régions (13, 15) qui sont espacées l'une de l'autre.

2. Membrane de Fresnel selon la revendication 1, dans laquelle l'encre de protection contre la lumière s'étend sur une largeur de 0,8 mm à 1,2 mm.

3. Membrane de Fresnel selon la revendication 1 ou 2, dans laquelle l'encre de protection contre la lumière divise la membrane de Fresnel en une région d'émission optique et une région de réception optique qui sont espacées l'une de l'autre, et l'encre de protection contre la lumière entoure la région d'émission optique.

4. Membrane de Fresnel selon l'une quelconque des revendications 1 à 3, dans laquelle le trou traversant comprend une première rainure et une seconde rainure qui communiquent entre elles, la première rainure et la seconde rainure sont partiellement alignées dans la direction d'épaisseur de la membrane de Fresnel, chacune de la première rainure et de la seconde rainure s'étend pour former un cercle fermé, et l'encre de protection contre la lumière est remplie dans la première rainure et la seconde rainure.

5. Membrane de Fresnel selon l'une quelconque des revendications 1 à 3, dans laquelle le trou traversant comprend une première rainure et une seconde rainure qui sont agencées dans la direction d'épaisseur de la membrane de Fresnel et qui communiquant entre elles, chacune de la première rainure et de la seconde rainure s'étend pour former un cercle fermé, l'encre de protection contre la lumière est remplie dans la première rainure et un matériau obtenu après qu'un matériau du substrat a subi une ablation laser se trouve dans la seconde rainure.

6. Membrane de Fresnel selon l'une quelconque des revendications 1 à 5, dans laquelle le substrat et les dents de Fresnel sont formés d'un seul tenant.

7. Membrane de Fresnel selon l'une quelconque des revendications 1 à 6, dans laquelle le substrat et les dents de Fresnel sont tous deux des matériaux plastiques transparents.

8. Procédé de production de membrane de Fresnel (100, 300, 400, 500), comprenant :
la fourniture d'une membrane de base transparente (110) ;
la formation de dents de Fresnel (30) sur une surface de la membrane de base transparente, de sorte que la membrane de base transparente est formée en un substrat (10) et les dents de Fresnel situées sur une surface du substrat ;
la réalisation d'une gravure sur le substrat et les dents de Fresnel, pour former un trou traversant (11) qui traverse le substrat et les dents de Fresnel dans une direction d'épaisseur, dans lequel le trou traversant s'étend pour former un cercle fermé ; et
le remplissage d'une encre de protection contre la lumière (50) dans le trou traversant.

9. Procédé de production de membrane de Fresnel selon la revendication 8, dans lequel
la réalisation d'une gravure sur le substrat et les dents de Fresnel pour former un trou traversant et le remplissage d'une encre de protection contre la lumière dans le trou traversant comprennent :
la réalisation d'une gravure sur le substrat et les dents de Fresnel pour former une première rainure, dans lequel la première rainure s'étend pour former un cercle fermé, et une profondeur de la première rainure est inférieure à une épaisseur totale du substrat et des dents de Fresnel ;
le remplissage de l'encre de protection contre la lumière dans la première rainure ;
la réalisation d'une gravure sur le substrat et les dents de Fresnel pour former une seconde rainure, dans lequel la seconde rainure est au moins partiellement alignée avec la première rainure et s'étend pour former un cercle fermé, et la seconde rainure communique avec la première rainure pour former le trou traversant ; et
le remplissage de l'encre de protection contre la lumière dans la seconde rainure.

10. Procédé de production de membrane de Fresnel selon la revendication 8, comprenant : la réalisation d'un gaufrage pour former une première rainure tout en réalisant un gaufrage sur la surface de la membrane de base pour former les dents de Fresnel, dans lequel la première rainure s'étend pour former un cercle fermé, et une profondeur de la première rainure est inférieure à une épaisseur totale du substrat et des dents de Fresnel ;
le remplissage de l'encre de protection contre la lumière dans la première rainure ;
la réalisation d'une gravure sur le substrat et les dents de Fresnel pour former une seconde rainure, dans lequel la seconde rainure est au moins partiellement alignée avec la première rainure et s'étend pour former un cercle fermé, et la seconde rainure communique avec la première rainure pour former le trou traversant ; et
le remplissage de l'encre de protection contre la lumière dans la seconde rainure.

11. Procédé de production de membrane de Fresnel selon la revendication 8, dans lequel la réalisation d'une gravure sur le substrat et les dents de Fresnel pour former un trou traversant et le remplissage d'une encre de protection contre la lumière dans le trou traversant comprennent :
la réalisation d'une gravure sur le substrat et les dents de Fresnel pour former une première rainure, dans lequel la première rainure s'étend pour former un cercle fermé, et une profondeur de la première rainure est inférieure à une épaisseur totale du substrat et des dents de Fresnel ;
le remplissage de l'encre de protection contre la lumière dans la première rainure ; et
la réalisation d'une ablation laser sur une région qui est du substrat et qui est alignée avec l'encre de protection contre la lumière dans la direction d'épaisseur du substrat, pour former une seconde rainure, dans lequel un matériau obtenu après qu'un matériau du substrat a subi une ablation laser se trouve dans la seconde rainure, et la seconde rainure est alignée avec la première rainure et s'étend pour former un cercle fermé.

12. Procédé de production de membrane de Fresnel selon l'une quelconque des revendications 9 à 11, dans lequel la profondeur de la première rainure est égale à la moitié de l'épaisseur du substrat et des dents de Fresnel, et une profondeur de la seconde rainure est égale à la moitié de l'épaisseur du substrat et des dents de Fresnel.

13. Procédé de production de membrane de Fresnel selon la revendication 8, dans lequel la réalisation d'une gravure sur le substrat et les dents de Fresnel pour former un trou traversant et le remplissage d'une encre de protection contre la lumière dans le trou traversant comprennent :
la réalisation d'une gravure sur le substrat et les dents de Fresnel pour former un premier trou traversant, dans lequel le premier trou traversant traverse le substrat et les dents de Fresnel ;
le remplissage de l'encre de protection contre la lumière dans le premier trou traversant ;
la réalisation d'une gravure sur le substrat et les dents de Fresnel pour former un second trou traversant, dans lequel le second trou traversant traverse le substrat et les dents de Fresnel, et le second trou traversant communique avec le premier trou traversant pour former un cercle fermé ; et
le remplissage de l'encre de protection contre la lumière dans le second trou traversant.

14. Module de détection optique, comprenant une membrane de Fresnel selon l'une quelconque des revendications 1 à 7, et un élément émetteur de lumière et un capteur photoélectrique qui sont disposés sur une face qui est de la membrane de Fresnel qui est opposée aux dents de Fresnel, dans lequel l'encre de protection contre la lumière divise la membrane de Fresnel en une région d'émission optique et une région de réception optique qui sont espacées l'une de l'autre, l'élément émetteur de lumière est disposé en alignement avec la région d'émission optique de la membrane de Fresnel, et le capteur photoélectrique est disposé en alignement avec la région de réception optique de la membrane de Fresnel.

15. Dispositif habitronique, comprenant le module de détection optique selon la revendication 14.
